# EUROPEAN PATENT SPECIFICATION

(11) **EP 2 720 755 B1**
(45) Date of publication and mention of the grant of the patent: **16.08.2017**
(21) Application number: 12803202.6
(22) Date of filing: 18.06.2012
(51) Int. Cl.: A61Q 5/12

(54) **COMPOSITIONS FOR DEPOSITING AGENTS USING HIGHLY VOLATILE SILICONE SOLVENTS**
ZUSAMMENSETZUNGEN ZUR ABLAGERUNG VON WIRKSTOFFEN MITTELS LEICHTFLÜCHTIGER SILIKONLÖSUNGSMITTEL
COMPOSITIONS POUR LE DÉPÔT D'AGENTS EMPLOYANT DES SOLVANTS DE SILICONE HAUTEMENT VOLATILS

(30) Priority: 18.06.2011 US 201161498553 P
(43) Date of publication of application: 23.04.2014
(73) Proprietor: Thompson Cooper Laboratories, LLC, Horsham, Pennsylvania 19044 (US)
(72) Inventor: COOPER, Eugene R, Berwyn, Pennsylvania 19312 (US); THOMPSON, Eric R, Horsham, Pennsylvania 19044 (US)
(74) Representative: Lee, Nicholas John
(86) International application number: PCT/US2012/042993
(87) International publication number: WO 2012/177560

(56) References cited:
- EP-A1- 2 077 123
- US-A- 5 916 548
- US-A- 5 916 548
- US-A1- 2003 232 030
- US-A1- 2003 235 553
- US-A1- 2006 211 820
- US-A1- 2007 037 732
- US-A1- 2007 037 732
- US-A1- 2008 226 584
- US-A1- 2009 041 694
- US-A1- 2009 041 694
- US-A1- 2009 186 943
- US-A1- 2010 310 492
- US-A9- 2004 192 845
- John Wiley and sons: "Organic Chemistry, Second Edition", 2003 page 337,
- "Silicone fluids: stable inert media", 2003, Gelest pages 1-25,

## Description

### Field of the Invention

The invention relates to the field of quickly and efficiently depositing germicides onto skin and other biological surfaces using highly volatile silicone compositions, which compositions are capable of depositing germicides, optionally in combination with skin conditioning and/or protective agents and sunscreen agents.

### Background of the Invention

The vehicles for application of materials to skin overwhelmingly tend to be in the form of aqueous-based systems. In such systems the materials to be applied are either in solution or suspension and can be thickened to create gels, lotions, and creams. Although water is volatile, it takes considerable time to evaporate, especially for creams. Such aqueous-based systems require the use of preservatives and can require considerable time to "rub-in". The least common application system for skin is an ointment or paste, which is messy and leaves a residual layer of viscous, tacky material. There is a need to deliver materials to skin in such a way as to avoid preservatives, odors (such as alcohols), and the potential for cooling, burning, and stinging (such as is found with alcohols), substantially free of water. There is also a need to complete the application process in a short time, such as in a few seconds, and to apply materials in such a way as to leave the skin feeling smooth and not tacky.

Sanitizer products are known in the art which sanitize skin and a variety of surfaces to render them germ-free. Germicidal products range from spray solutions to bactericidal hand soaps. These products evidence the desire of people to maintain germ-free environments, minimizing exposure to harmful bacteria and other microorganisms, by depositing chemicals on surfaces which kill such organisms. In addition to the desire to sanitize such surfaces as kitchen counters, bathroom sinks, toilet seats, and the like, it would also be desirable to retain the germicidal chemical on the surface to maintain sanitizing character to the surface for an extended period of time. There are numerous products (see for example http://www.artandopinion.com/2007_v6_n6/glaser-triclosan.htm) that contain the chemical triclosan, and agents such as tea tree oil or benzalkonium chloride, which are known to kill microorganisms. Perhaps the most common surface sanitizer is one that contains alcohol in a gel or spray formulation. Alcohol-based products tend to dry the skin, they can burn and sting, and they do not retain antimicrobial activity. These and other products also require considerable time for "rubbing in" or "drying" of the formulation.

There is a need in the art to deposit antimicrobial agents on surfaces not only to sanitize the surface of harmful microorganisms, but advantageously to retain such agent on the surface in order to provide sanitizing conditions to the surface for an extended period of time.

Skin conditioning agents are typically applied as lotions or creams, which are aqueous based and require preservatives and "rub in" to evaporate the water. There is a need to apply conditioning materials to skin without water and which requires only a few seconds and can leave conditioning agents on skin. Likewise, there is a need to apply such agents as sunscreens in highly retentive films in a matter of seconds.

US2006/0211820 discloses liquid compositions that comprise siloxane-containing polymer, volatile polydimethylsiloxane, aliphatic hydrocarbon and an adjuvant, which optionally is a medicament, which optionally is benzalkonium chloride.

The art is in need of superior compositions, free of water, and free of the problems of today's known film-depositing compositions.

### Summary of the Invention

In a first aspect, the present invention provides a nonaqueous composition comprising
(a) an alkyl quaternary ammonium germicide,
(b) a highly volatile silicone solvent selected from the group consisting of hexamethyl disiloxane and octamethyl trisiloxane,
(c) a single film-forming polymer which is polydimethylsiloxane having a viscosity of
   0.05 m²/s (50,000 cSt) or greater,
(d) less than 5% by weight ethanol or isopropanol.

The alkyl quaternary ammonium germicide may be benzalkonium chloride or benzethonium chloride. The composition may further comprise a skin conditioner/protectant or a sunscreen. The composition may further comprise a local anesthetic, an insect repellent, a fragrance, a colorant, a drug, or a vitamin. The composition may be in a form selected from the group consisting of a solution, a dispersion, a gel, and a semi-solid.

In a second aspect, the present invention provides a method for disinfecting a surface, comprising applying the composition of the first aspect of the invention to the surface, wherein the method is not a method for treatment of the human or animal body by surgery or therapy practised on the human or animal body.

The disadvantages of both aqueous based systems and alcohol based systems can be avoided by using a nonaqueous delivery system based on the highly volatile silicone fluids hexamethyldisiloxane or octamethyltrisiloxane as the highly volatile carrier for a myriad of materials. Such systems can be used in accordance with the invention to deliver an alkyl quaternary ammonium germicide , optionally in combination with skin conditioning agents, retentive polymers, skin feel agents, skin protective agents, and other materials in a fluid that spreads and evaporates in a few seconds. Such systems can be sprayed onto skin without rubbing, which is a particular advantage for persons applying materials to legs and feet and where touching is undesirable. Elderly persons, for example, have particular difficulty bending over and rubbing topical products on their legs and feet, and these systems allow for the elderly to apply beneficial agents such as sanitizers and skin conditioning agents rapidly and without rubbing or having to reach their feet. A single film-forming polymer which is polydimethylsiloxane having a viscosity of 50,000 cSt or greater can be applied as a skin protectant for diaper rash prevention in a few seconds, leaving the skin smooth and non-tacky, as contrasted to the highly viscous and sticky pastes that are commonly used. Compositions of the invention can advantageously be applied to broken skin such as abrasions without pain and touching. Agents can thereby be delivered to the skin in a few seconds leaving behind the agents or a thin film containing the desired agent.

The invention allows delivery of an alkyl quaternary ammonium germicide to a surface with a system that does not contain high levels of alcohol, and which can be applied within 5 seconds or less. Furthermore this system can provide for the retention of the germicide as well as extended protection for the surface or skin, and it may contain other agents for conditioning and lubricating skin as well as other cosmetically desired ingredients. Such a system may be a non-viscous fluid that can be sprayed onto a surface, that deposits within a few seconds a thin film of sanitizing formulation with or without rubbing. Thus it may easily and rapidly be used for application to feet and other surfaces which are hard to reach. The system can also be applied to a variety of surfaces, such as leather, wood, clothing, and the like, without the disadvantages associated with aqueous and alcohol-based products. The present invention thereby overcomes the problems of known sanitizer compositions. Particularly, the invention permits the delivery of an alkyl quaternary ammonium germicide to a surface wherein the composition avoids the disadvantages of known sanitizer compositions, (e.g., does not burn, sting, or dry the skin), and which can be applied quickly (e.g., within 5 seconds or less) without the need to "rub in". Furthermore the compositions of the invention are capable of providing for the retention of the alkyl quaternary ammonium germicide on the surface (skin or other surfaces) and thereby maintain sanitizing protection for the skin and other surfaces.

The invention includes compositions and methods for delivering an alkyl quaternary ammonium germicide (e.g., benzalkonium chloride) to a surface to not only sanitize the surface but to provide sanitization for an extended period of time. The agent optionally may be applied in a thin film; more specifically, the invention provides the ability to deliver such a film via a liquid that can be sprayed or placed on a surface by using a highly volatile solvent such that the film can be formed/spread quickly and conveniently. For example, such a system can be sprayed onto the skin, on household surfaces, or even animals (and either be spread or not) and it will dry within a few seconds to provide a thin film that is invisible but yet which can provide extended sanitization for several hours.

In one embodiment, the invention provides a composition in which the alkyl quaternary ammonium germicide is benzalkonium chloride. Spraying a small quantity of such a sanitizer composition onto skin will deposit within a few seconds a thin film containing benzalkonium chloride such that the skin retains its smooth feel despite the fact that the thin sanitizing film remains thereon. This system provides a rapid and convenient way to provide immediate and long-lasting sanitization protection. Other suitable alkyl quaternary ammonium germicides include benzethonium chloride.

Other optional desirable ingredients may be included and deposited in a like manner and in combination. Thus, in some aspects, the compositions of the invention may contain other additional ingredients such as skin conditioners, local anesthetics (e.g., lidocaine), sunscreens, insect repellents, fragrances, colorants, drugs, nutrients, and vitamins. Such a sanitizing composition is generally a non-viscous fluid that can be sprayed onto a surface, depositing within a few seconds a very thin film of sanitizing formulation without rubbing, although rubbing is optional. Thus it may easily and rapidly be used for application to feet and other hard to reach surfaces. The compositions of the invention may advantageously be applied to a wide variety of ordinary items, such as leather, wood, clothing, etc., without the problems associated with other aqueous and alcohol-based products.

The film-forming polymer is polydimethylsiloxane (0.05 m²/s (50,000 cSt) or greater). These highly viscous silicone polymers are highly sticky and tacky but surprisingly when applied according to the invention, the skin feels lubricated and the film is highly retentive and protective, particularly from water-soluble irritants.
The skin conditioner/protectant may be selected from Croda's Liquid Medilan™ Ultra (lanolin oil (LO)), petrolatum (WP), Croda's Medilan™ USP and Medilan Ultra-lanolins, Dow Corning® Soy wax, Dow Corning® Cosmetic wax, Shea butter, dodecamethylpentasiloxane (2.0cSt), Dow Corning® ST-Cyclomethicone5-NF, Dow Corning® 9040, and ST Elastomer 10.

The sunscreen may be octyl salicylate.

Optional ingredients may be present in the composition, such as those selected from a local anesthetic, an insect repellent, a fragrance, a colorant, a drug, and a vitamin.

The composition may be in a functionally useful form, such as a solution, a dispersion, a gel, and a semi-solid.

Disclosed herein is a method of applying a composition of the invention by administering the composition to the desired surface, such as the skin. In some aspects, the composition is in a liquid form in a spray bottle, while other forms are readily administered by direct application.

Such aspects of the invention are generally free of water.

These and other objects are achieved through the present invention as exemplified and further described in the Detailed Description of the invention below.

### Detailed Description of the Invention

The compositions of the invention comprise an effective amount of an alkyl quaternary ammonium germicide mixed with a highly volatile silicone solvent selected from hexamethyldisiloxane and octamethyltrisiloxane, a film-forming polymer which is polydimethylsiloxane having a viscosity of 0.05 m²/s (50,000 cSt) or greater, and less than 5% by weight ethanol or isopropanol. The solvent and polymer permit the application of the germicide such that the solvent rapidly evaporates, leaving a thin film comprised of the polymer and the germicide.

Benzalkonium chloride, or other alkyl quaternary ammonium germicide, can be dissolved in a highly volatile silicone solvent selected from hexamethyldisiloxane (HDS) and/or octamethyltrisiloxane. Typical levels of benzalkonium chloride are 0.13% or less. A small amount (less than 3%) of a low molecular weight alcohol selected from isopropyl alcohol and ethanol is used to help solubilize polar germicides like benzalkonium chloride.

In order to form an invisible but retentive film which maintains the germicidal agent on the surface, a film-forming polymer which is polydimethylsiloxanse having a viscosity of 0.05 m²/s (50,000 cSt) is included in the composition.

Certain skin conditioning agents that are somewhat retentive include Croda's Liquid Medilan™ Ultra (lanolin oil (LO)), petrolatum (WP), Croda's Medilan™ USP and Medilan™ Ultra-lanolins, Dow Corning® Soy wax, and Dow Corning® Cosmetic wax. Certain "skin feel" agents are also potentially retentive, such as moderately volatile silicones like dodecamethylpentasiloxane (2.0 x 10⁻⁶ m²/s (2.0cSt)), decamethyltetrasiloxane, and Dow Corning® ST-Cyclomethicone5-NF and gels such as Dow Corning® 9040 and ST Elastomer 10.

Local anesthetics, sunscreens, insect repellents, fragrances, colorants, drugs, nutrients, and vitamins may additionally be included.

### Highly volatile silicone solvents

Hexamethyldisiloxane and octamethyltrisiloxane are the highly volatile silicone solvents used in the present invention.

### Film-forming polymers

It is desired to maintain the activity of the germicide for an extended period of time following its application. Accordingly, a film-forming polymer is included in the composition which, upon volatilization of the silicone solvent, (i.e., as the applied composition dries) forms a thin film, substantially invisible and substantially undetectable to touch, which film contains the still-active germicide. Such film-forming polymer is polydimethylsiloxane (0.05 m²/s (50,000 cSt) or greater The compositions of the invention have a single film-forming polymer.

### Germicidal agents

While many of the embodiments and examples recited herein use benzalkonium chloride as the germicidal agent, such agents include all alkyl quaternary ammonium germicides, including benzethonium chloride. Certain quaternary ammonium germicides require alcohol to be added to the composition in order to facilitate their solubilization in the highly volatile silicone solvent.

### Skin conditioners

The composition of the invention may comprise agents directed to skin care in general included in the formulation. Thus, for example, the composition may include such agents as protecting/conditioning materials such as Croda's Liquid Medilan™ Ultra (lanolin oil (LO)), petrolatum (WP), Croda's Medilan™ USP and Medilan Ultra-lanolins, Dow Corning® Soy wax, and Dow Corning® Cosmetic wax, Shea butter and "skin feel" agents such as low volatile silicones like dodecamethylpentasiloxane (2.0 x 10⁻⁶ m²/s (2.0cSt))
and Dow Corning® ST-Cyclomethicone5-NF and gels such as Dow Corning® 9040 and ST Elastomer 10. The compositions of the invention may have a single conditioner, or may comprise a combination of two or more such conditioners. The compositions may be solutions or dispersions, and the dispersions may be formed in a variety of ways including but not limited to making a solution at higher temperatures followed by agitation/high shear mixing as the solution cools. Additionally one may employ methods such cavitation/microfluidization to create nano sized dispersions.

### Other agents and optional components

In some embodiments, optional components include local anesthetics (e.g., benzocaine), sunscreens (e.g., octyl salicylate), insect repellents (e.g., N,N,-Diethyl-meta-toluamide (DEET)), fragrances (e.g., lavender oil), colorants (e.g., beet root extract), drugs (e.g, retinol), , and vitamins (e.g., nicotinamide). The compositions of the invention may have a single such optional component, or may comprise a combination of two or more such optional components.

### Forms of the compositions of the invention

In some embodiments, the composition of the invention is in the form of a liquid, generally applied in a spray bottle or other application device. In other embodiments, the invention's compositions may be formulated in a gel form, for direct application to the skin. Such gels may be prepared by using a gelling agent, such as Dow Corning® 9040 and ST Elastomer 10 added to the composition.

### Uses of the compositions of the invention

Essentially any surface to be sanitized or coated is an appropriate surface for the compositions of the invention to be applied to. The embodiments of the invention with skin protectants and conditioners are highly suitable for use on skin surfaces, whether it be the hands, face, feet, etc. Such embodiments are also well-suited for other biological surfaces. Germicidal compositions of the invention can be applied to counters, doorknobs, leather goods, and the like; where the surface to be treated is not skin, embodiments of the invention without skin protectants and conditioners are quite suitable for extended sanitization.

The intended scope of the invention is only limited by the claims appended hereto.

### Examples

The present invention will be further understood by reference to the following nonlimiting examples.

### Germicidal compositions

### Example 1: germicidal composition

On a weight basis 0.13% benzalkonium chloride, 2.5% isopropyl alcohol, 1.3% polydimethyl siloxane (0.3 m²/s (300,000 cSt))
and 96.07% hexamethyldisiloxane were mixed to form a clear solution. This solution when sprayed onto skin or other surface can be spread and dried in a few seconds to leave a smooth film containing benzalkonium chloride,

### Example 2: germicidal composition

On a weight basis 0.13% benzalkonium chloride, 1.25% ethyl alcohol, 0.8% polydimethyl siloxane (0.3 m²/s (300,000 cSt))
0.5% Dow Corning® 9040, and 97.32% sprayed onto skin or other surface can be spread and dried in a few seconds to leave a smooth film containing benzalkonium chloride.

### Reference Example 1: germicidal composition

On a weight basis 0.13% benzalkonium chloride, 2.5% isopropyl alcohol, 1% Dow Corning® Cosmetic or Soy Wax, and 96.37% hexamethyldisiloxane were mixed to form a clear solution upon heating. This mixture became turbid upon cooling to room temperature and when sprayed onto skin or other surface can be spread and dried in a few seconds to leave a smooth film containing benzalkonium chloride.

### Example 3: germicidal composition

On a weight basis 0.13% benzalkonium chloride, 1.25% ethyl alcohol, 1.0% polydimethyl siloxane (0.3 m²/s (300,000 cSt))
1.0% Croda Liquid Medilan™ Ultra, 3% Dow Corning® ST-Cyclomethicone 5-NF, and 93.62% hexamethyldisiloxane were mixed to form a clear solution. This solution when sprayed onto skin or other surface can be spread and dried in a few seconds to leave a smooth film containing benzalkonium chloride.

### Reference Example 2: germicidal composition

On a weight basis 0.13% benzalkonium chloride, 1.25% ethyl alcohol, 2.3% Croda Liquid Medilan™ Ultra, 0.7% Dow Corning® 9040,2% Dow Corning ST-Cyclomethicone 5-NF, and 93.62% hexamethyldisiloxane were mixed to form a clear solution. This slightly turbid mixture when sprayed onto skin or other surface can be spread and dried in a few seconds to leave a smooth film containing benzalkonium chloride.

### Reference Example 3: germicidal composition

On a weight basis 0.13% benzalkonium chloride, 1.25% ethyl alcohol, 1.0% Croda Liquid Medilan™ Ultra, 4% Dow Corning® ST-Cyclomethicone 5-NF, and 93.62% hexylmethyldisiloxane were mixed to form a clear solution. This solution when sprayed onto skin or other surface can be spread and dried in a few seconds to leave a smooth film containing benzalkonium chloride.

### Reference Example 4: germicidal composition

On a weight basis 0.13% benzalkonium chloride, 1.25% ethyl alcohol, 2.0% Croda Liquid Medilan™ Ultra, 3% Dow Corning® ST-Cyclomethicone 5-NF, and 93.62% hexylmethyldisiloxane were mixed to form a clear solution. This solution when sprayed onto skin or other surface can be spread and dried in a few seconds to leave a smooth film containing benzalkonium chloride.

### Reference Example 5: germicidal composition

On a weight basis 0.13% benzalkonium chloride, 1.25% ethyl alcohol, 3.0% Croda Liquid Medilan™ Ultra, 2% Dow Corning® ST-Cyclomethicone 5-NF, and 93,62% hexamethyldisiloxane were mixed to form a clear solution. This solution when sprayed onto skin or other surface can be spread and dried in a few seconds to leave a smooth film containing benzalkonium chloride.

### Reference Example 6: germicidal composition

On a weight basis 0.13% benzalkonium chloride, 1.25% ethyl alcohol, 2.0% Croda Liquid Medilan™ Ultra, 3% octamethyltrisiloxane, and 93.62% hexamethyldisiloxane were mixed to form a clear solution. This solution when sprayed onto skin or other surface can be spread and dried in a few seconds to leave a smooth film containing benzalkonium chloride.

### Example 4. : germicidal composition

On a weight basis 0.13% benzalkonium chloride, 1.25% ethyl alcohol, 5% polydimethyl siloxane (0.3 m²/s (300,000 cSt))
0.2% lidocaine, and 93.42% hexamethyldisiloxane were mixed to form a clear solution. This solution can be sprayed onto a light wound or abrasion to prevent infection, control pain, and provide a protective coating.

### Example 5: germicidal composition

On a weight basis 0.13% benzalkonium chloride, 2.5% isopropyl alcohol, 1.3% polydimethyl siloxane (0.3 m²/s (300,000 cSt))
0.2% lidocaine, and 95.87% hexamethyldisiloxane were mixed to form a clear solution. This solution can be sprayed onto a light would or abrasion to prevent infection, control pain, and provide a protective coating.

### Reference Example 7: germicidal composition

On a weight basis 0.13% benzalkonium chloride, 1.25% ethyl alcohol, 0.2% lidocaine, and 98.42% hexamethyldisiloxane were mixed to form a clear solution. This solution can be sprayed onto a light wound or abrasion to prevent infection, control pain, and provide a protective coating.

### Reference Example 8: germicidal composition

On a weight basis 0.13% benzalkonium chloride, 1.25% ethyl alcohol, and 98.62% hexamethyldisiloxane were mixed to form a clear solution. This solution can be sprayed onto a surface or wound where no residue is desired. Such an example would be for example to prevent infection in animal ears.

### Reference Example 9: germicidal composition

On a weight basis 0.13% benzalkonium chloride, 1.25% ethyl alcohol, 2.0% Croda Liquid Medilan™ Ultra, 3.0% octamethylcyclotetrasiloxane, and 93.62% octamethyltrisiloxane were mixed to form a clear solution. This solution when sprayed onto skin or other surface can be spread and dried in a few seconds to leave a smooth film containing benzalkonium chloride.

### Reference Example 10: germicidal composition

On a weight basis 0.13% benzalkonium chloride, 1.25% ethyl alcohol, 2.0% Croda Liqiud Medilan™ Ultra, 3.0% decamethylcyclopentasiloxane, and 93.62% hexamethyldisiloxane were mixed to form a clear solution. This solution when sprayed onto skin or other surface can be spread and dried in a few seconds to leave a smooth film containing benzalkonium chloride.

### Reference Example 11: germicidal composition

On a weight basis 0.13% benzalkonium chloride, 1.25% ethyl alcohol, 2.0% Croda Liquid Medilan™ Ultra, 3.0% octamethylcyclotetrasiloxane, 30% octamethyltrisiloxane, and 63.62% hexamethlydisiloxane were mixed to form a clear solution. This solution when sprayed onto skin or other surface can be spread and dried in a few seconds to leave a smooth film containing benzalkonium chloride.

### Nongermicidal compositions not within the scope of the invention

Skin conditioning and moisturizing, skin protectant, and wound healing compositions without antimicrobial agents are shown in the following reference example compositions, which do not contain water, do not burn or sting, can be sprayed on or applied by gentle rubbing, and can be used on the face as well. They leave the skin with a very smooth and non-sticky feel.

### Reference Example 12: skin conditioning compositions

The following dispersions of solid moisturizers in the highly volatile hexamethyldisiloxane were obtained by heating all ingredients till a clear solution was obtained and then gradual cooling with vigorous shaking as the dispersion forms:
12.1) By weight 10% Dow Corning® HY-3050 Soy Wax and 90% hexamethyldisiloxane were mixed and processed as described above to provide a low viscosity dispersion for rapid and even application to skin.
12.1 a) By weight 10% Dow Corning® ST-Wax30 and 90% hexamethyldisiloxane were mixed and processed as described above to provide a low viscosity dispersion for rapid and even application to skin.
12.1b) By weight 10% Medilan™ Ultra Lanolin and 90% hexamethyldisiloxane were mixed and processed as described above to provide a low viscosity dispersion for rapid and even application to skin.
12.2) By weight 20% Dow Corning® ST-Wax30 and 80% hexamethyldisiloxane were mixed and processed as described above to provide a low viscosity dispersion for rapid and even application to skin.
12.2a) By weight 20% Dow Corning® HY-3050 Soy Wax and 80% hexamethyldisiloxane were mixed and processed as described above to provide a low viscosity dispersion for rapid and even application to skin.
12.2b) By weight 20% Medilan™ Ultra Lanolin and 80% hexamethyldisiloxane were mixed and processed as described above to provide a low viscosity dispersion for rapid and even application to skin.
12.3) By weight 20% Dow Corning® ST-Wax30, 10% Dow Corning® ST-Elastomer 10, and 70% hexamethyldisiloxane were mixed and processed as described above to provide a low viscosity dispersion for rapid and even application to skin.
12.3a) By weight 20% Dow Corning® HY-3050 Soy Wax, 10% Dow Corning® ST-Elastomer 10, and 70% hexamethyldisiloxane were mixed and processed as described above to provide a low viscosity dispersion for rapid and even application to skin.
12.3b) By weight 20% Medilan™ Ultra Lanolin, 10% Dow Corning® ST-Elastomer 10, and 70% hexamethyldisiloxane were mixed and processed as described above to provide a low viscosity dispersion for rapid and even application to skin.
12.4) By weight 35% Medilan™ Ultra Lanolin, 20% Dow Corning® ST-Elastomer 10, and 45% hexamethyldisiloxane were mixed and processed as described above to provide a medium viscosity dispersion for rapid and even application to skin.
12.5) By weight 45% Medilan™ Ultra Lanolin, 20% Dow Corning® ST-Elastomer 10, and 35% hexamethyldisiloxane were mixed and processed as described above to provide a viscous dispersion for rapid and even application to skin.
12.6) By weight 20% Medilan™ Ultra Lanolin, 20% Dow Corning® ST-Cyclomethicone 5-NF, and 60% hexamethyldisiloxane were mixed and processed as described above to provide a low viscosity dispersion for rapid and even application to skin.
12.7) By weight 25% Liquid Medilan™ Ultra Lanolin, 20% Dow Corning® ST-Cyclomethicone 5-NF, 2.5% beeswax, and 52.5% hexamethyldisiloxane were mixed and processed as described above to provide a low viscosity dispersion for rapid and even application to skin.
12.8) By weight 25% Liquid Medilan™ Ultra Lanolin, 20% Dow Corning® ST-Cyclomethicone 5-NF, 2.5% Dow Corning® ST-Wax30, and 52.5% hexamethyldisiloxane were mixed and processed as described above to provide a low viscosity dispersion for rapid and even application to skin.
12.9) By weight 25% Liquid Medilan™ Ultra Lanolin, 20% Dow Corning® ST-Cyclomethicone 5-NF, Dow Corning® HY-3050 Soy Wax, and 52.5% hexamethyldisiloxane were mixed and processed as described above to provide a low viscosity dispersion for rapid and even application to skin.
12.10) By weight 25% Liquid Medilan™ Ultra Lanolin, 20% Dow Corning® ST-Cyclomethicone 5-NF, 5% Dow Corning® ST-Wax30, and 50% hexamethyldisiloxane were mixed and processed as described above to provide a low viscosity dispersion for rapid and even application to skin.

### Reference Example 13: skin conditioning compositions with additional ingredients

13.1) By weight 32% Liquid Medilan™ Ultra Lanolin, 3%Vitamin E, and 65% hexamethyldisiloxane were dissolved together to provide a low viscosity solution for rapid and even application to skin.
13.2) By weight 35% Liquid Medilan™ Ultra Lanolin, 20% Dow Corning® ST-Elastomer 10, and 45% hexamethyldisiloxane were dissolved together to provide a low viscosity hazy solution for rapid and even application to skin. (This product was given to a person who works in construction with bleeding dry hands with band aids, and after one week the hands were markedly improved with only two applications per day)
13.3) By weight 22% Liquid Medilan™ Ultra Lanolin, 3%Vitamin E, 10% Dow Corning® ST-Elastomer 10, and 65% hexamethyldisiloxane were dissolved together to provide a low viscosity hazy solution for rapid and even application to skin.
13.4) By weight 20% Liquid Medilan™ Ultra Lanolin, 20% Dow Corning® ST-Cyclomethicone 5-NF, and 60% hexamethyldisiloxane were mixed and processed as described above to provide a low viscosity solution for rapid and even application to skin.
13.5) By weight 32% Liquid Medilan™ Ultra Lanolin, 3%Vitamin E, 20% Dow Corning® ST-Cyclomethicone 5-NF, and 45% hexamethyldisiloxane were mixed and processed as described above to provide a medium viscosity solution for rapid and even application to skin.
13.6) By weight 35% Liquid Medilan™ Ultra Lanolin, 20% Dow Corning® ST-Cyclomethicone 5-NF, and 45% hexamethyldisiloxane were mixed and processed as described above to provide a medium viscosity solution for rapid and even application to skin.

### Reference Example 13a): Skin protectant compositions

13a.1) By weight 5% polydimethylsiloxane (0.3 m²/s (300,000 cSt)) and 95% hexamethyldisiloxane were mixed to give a clear solution that can be rapidly and evenly applied to lubricate and protect skin.
13a.2) By weight 5% polydimethylsiloxane (1 m²/s (1,000,000 cSt)) and 95% hexamethyldisiloxane were mixed to give a clear solution that can be rapidly and evenly applied to lubricate and protect skin.
13a.3) By weight 10% polydimethylsiloxane (0.3 m²/s (300,000 cSt)) and 90% hexamethyldisiloxane were mixed to give a clear solution that can be rapidly and evenly applied to lubricate and protect skin.
13a.4) By weight 10% polydimethylsiloxane (1 m²/s (1,000,000 cSt)) and 90% hexamethyldisiloxane were mixed to give a clear solution that can be rapidly and evenly applied to lubricate and protect skin.

### Reference Example 14: sunscreen compositions

14.1) Dissolve 5 grams octyl salicylate in 90 grams hexmethyldisiloxane and add 5 grams polydimethylsiloxane (0.3 m²/s (300,000 cSt)). Solution/mixture can be sprayed onto surface leaving a smooth, retentive film.
14.2) Dissolve 10 grams octyl salicylate in 80 grams hexmethyldisiloxane and add 10 grams polydimethylsiloxane (0.3 m²/s (300,000 cSt)). Solution/mixture can be sprayed onto surface leaving a smooth, retentive film.
14.3) Dissolve 5 grams octyl salicylate in 87 grams hexmethyldisiloxane, 3 grams cyclomethicone and add 5 grams polydimethylsiloxane (0.3 m²/s (300,000 cSt)). Solution/mixture can be sprayed onto surface leaving a smooth, retentive film.
14.4) Dissolve 3 grams avobenzone in 92 grams hexmethyldisiloxane and add 5 grams polydimethylsiloxane (0.3 m²/s (300,000 cSt)). Solution/mixture can be sprayed onto surface leaving a smooth, retentive film.
14.5) Dissolve 5 grams octyl methoxycinnamate in 90 grams hexmethyldisiloxane and add 5 grams polydimethylsiloxane (0.3 m²/s (300,000 cSt)). Solution/mixture can be sprayed onto surface leaving a smooth, retentive film.
14.6) Dissolve 7.5 grams octyl methoxycinnamate in 84.5 grams hexmethyldisiloxane and add 8 grams polydimethylsiloxane (0.3 m²/s (300,000 cSt)). Solution/mixture can be sprayed onto surface leaving a smooth, retentive film.

## Claims

1. A nonaqueous composition comprising
(a) an alkyl quaternary ammonium germicide,
(b) a highly volatile silicone solvent selected from the group consisting of hexamethyl disiloxane and octamethyl trisiloxane,
(c) a single film-forming polymer which is polydimethylsiloxane having a viscosity of 0.05 m²/s (50,000 cSt) or greater, and
(d) less than 5% by weight ethanol or isopropanol.

2. The composition of claim 1, wherein the alkyl quaternary ammonium germicide is benzalkonium chloride or benzethonium chloride.

3. The composition of any preceding claim, further comprising a skin conditioner/protectant or a sunscreen.

4. The composition of any preceding claim, further comprising a local anesthetic, an insect repellent, a fragrance, a colorant, a drug, or a vitamin.

5. The composition of any preceding claim, wherein the composition is in a form selected from the group consisting of a solution, a dispersion, a gel, and a semi-solid.

6. A method for disinfecting a surface, comprising applying the composition of any one of claims 1-5 to the surface, wherein the method is not a method for treatment of the human or animal body by surgery or therapy practised on the human or animal body.

## Patentansprüche

1. Nicht-wässrige Zusammensetzung, umfassend
(a) ein Germizid (keimtötendes Mittel) aus quaternärem Alkylammonium,
(b) ein hochgradig flüchtiges Silikon-Lösungsmittel, das ausgewählt ist aus der Gruppe bestehend aus Hexamethyldisiloxan und Octamethyltrisiloxan,
(c) ein einzelnes filmbildendes Polymer, welches Polydimethylsiloxan mit einer Viskosität von 0,05 m²/s (50.000 cSt) oder größer ist, und
(d) weniger als 5 Gewichts-% Ethanol oder Isopropanol.

2. Die Zusammensetzung nach Anspruch 1, wobei das quaternäre Alkylammonium-Germizid Benzalkoniumchlorid oder Benzethoniumchlorid ist.

3. Die Zusammensetzung nach jedem vorangehenden Anspruch, außerdem umfassend ein Hautpflegemittel/-schutzmittel oder ein Sonnenschutzmittel.

4. Die Zusammensetzung nach jedem vorangehenden Anspruch, außerdem umfassend ein Lokalanästhetikum, ein Insektenschutzmittel, einen Duftstoff, einen Farbstoff, einen Wirkstoff oder ein Vitamin.

5. Die Zusammensetzung nach jedem vorangehenden Anspruch, wobei die Zusammensetzung in einer Form vorliegt, die ausgewählt ist aus der Gruppe bestehend aus einer Lösung, einer Dispersion, einem Gel und einer halbfesten Substanz.

6. Verfahren zum Desinfizieren einer Oberfläche, umfassend das Auftragen der Zusammensetzung nach einem der Ansprüche 1-5 auf die Oberfläche, wobei das Verfahren nicht ein Verfahren zur Behandlung des menschlichen oder tierischen Körpers mittels an dem menschlichen oder tierischen Körper ausgeübter Chirurgie oder Therapie ist.

## Revendications

1. Composition non-aqueuse contenant :
a) un germicide alkylammonium quaternaire,
b) un solvant silicone hautement volatile choisi dans le groupe constitué de l'hexaméthyl disiloxane et de l'octaméthyl trisiloxane,
c) un polymère formant un film unique qui est un polydiméthylsiloxane ayant une viscosité d'au moins 0,05 m²/s (50 000 cSt), et
d) moins de 5 % en poids d'éthanol ou d'isopropanol.

2. Composition selon la revendication 1, dans laquelle le germicide alkylammonium quaternaire est du chlorure de benzalkonium ou du chlorure de benzéthonium.

3. Composition selon l'une quelconque des revendications précédentes, contenant en outre un conditionneur cutané / protecteur ou un écran solaire.

4. Composition selon l'une quelconque des revendications précédentes, contenant en outre un anesthésique local, un répulsif contre les insectes, un parfum, un colorant, un médicament ou une vitamine.

5. Composition selon l'une quelconque des revendications précédentes, la composition étant sous une forme choisie dans le groupe constitué d'une solution, d'une dispersion, d'un gel et d'un semi-solide.

6. Procédé de désinfection d'une surface, comprenant l'application de la composition selon l'une quelconque des revendications 1 à 5 sur la surface, le procédé n'étant pas un procédé de traitement du corps humain ou animal par chirurgie ou thérapie pratiquée sur le corps humain ou animal.
